(19) Europäisches Patentamt
European Patent Office
Office européen des brevets

(11) Numéro de publication : **0 340 117 B1**

(12) **FASCICULE DE BREVET EUROPEEN**

(45) Date de publication du fascicule du brevet : **08.07.92 Bulletin 92/28**

(51) Int. Cl.$^5$ : **C07C 45/46,** C07C 49/784, C07C 49/84, B01J 27/12, C07C 323/22

(21) Numéro de dépôt : **89420046.8**

(22) Date de dépôt : **13.02.89**

(54) **Procédé de diacylation de composés comportant deux noyaux aromatiques.**

(30) Priorité : **18.02.88 FR 8802192**

(43) Date de publication de la demande : **02.11.89 Bulletin 89/44**

(45) Mention de la délivrance du brevet : **08.07.92 Bulletin 92/28**

(84) Etats contractants désignés : **AT BE CH DE ES FR GB GR IT LI LU NL SE**

(56) Documents cités :
**EP-A- 0 069 598**
**EP-A- 0 084 742**
**EP-A- 0 178 184**
**EP-A- 0 189 266**

(56) Documents cités :
**EP-A- 0 193 475**
**EP-A- 0 199 661**
**HOUBEN-WEYL, 4ième édition, 1973, tome VII/2A, pages 21,62, Georg Thieme Verlag, Stuttgart, DE; "Methoden der organischen-Chemie"**

(73) Titulaire : **ELF ATOCHEM S.A.**
**4 & 8, Cours Michelet La Défense 10**
**F-92800 Puteaux (FR)**

(72) Inventeur : **Devic, Michel**
**27, Chemin des Fonds**
**F-69110 Sainte-Foy-les-Lyon (FR)**
Inventeur : **Kervennal, Jacques**
**134, rue E. Locard**
**F-69005 Lyon (FR)**

**EP 0 340 117 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de diacylation de composés ayant deux noyaux aromatiques dans leur molécule, de formule générale :

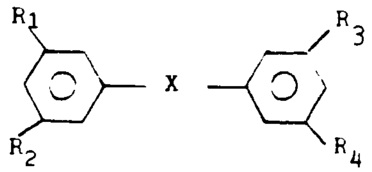

(I)

dans laquelle

X désigne un atome d'oxygène ou de soufre, un groupe

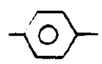

ou $-CH_2-$.

$R_1$, $R_2$, $R_3$, $R_4$, identiques ou différents, représentent un atome d'hydrogène ou un groupe alkyl ayant de 1 à 4 atomes de carbone.

Par diacylation on entend ici comme dans tout ce qui suit, la transformation d'un composé de formule générale (I) en un composé de formule générale :

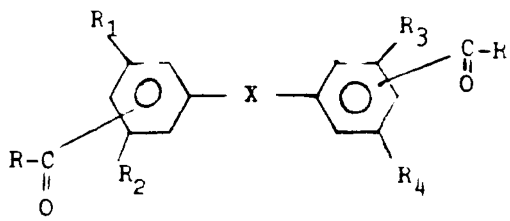

dans laquelle

X, $R_1$, $R_2$, $R_3$, $R_4$ ont la même définition que dans (I),

R est défini comme $R_1$, $R_2$, $R_3$, $R_4$,

à l'aide d'un agent d'acylation de formule générale :

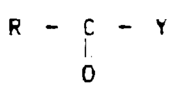

(III)

dans laquelle

Y est un atome d'halogène, un groupe -OH, -OR ou

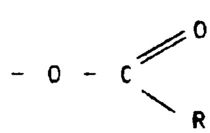

,

R étant chaque fois défini comme dans (II).

Des exemples d'agents d'acylation ainsi définis sont donnés par exemple dans la demande de brevet européen EP-069597.

Les composés de formule (II) sont utilisés dans l'industrie comme intermédiaires, par exemple pour accéder à des polymères de type polyester. C'est le cas en particulier de ceux pour lesquels R se trouve en position 4 et 4′, comme le diacétyl-4,4′-diphényléther :

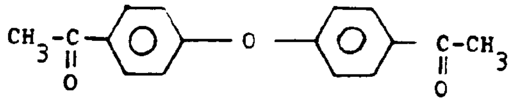

Il est connu d'acyler le diphényléther, pour former par exemple la phénoxy-4-acétophénone :

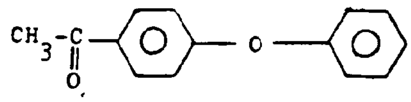

comme le décrit par exemple la demande de brevet européen déjà citée.

Cette même demande enseigne que l'acylation en présence d'acide fluorhydrique HF exclut pratiquement la diacylation, permise cependant par le chlorure d'aluminium $AlCl_3$.

Il est connu que les inconvénients liés à l'emploi de ce dernier en limitent largement l'intérêt industriel.

En outre, dans EP-0084742, il est décrit que le composé de formule (I) peut dériver du diphénylether. Mais celle-ci enseigne que l'acylation d'un tel composé conduit à un dérivé monoacylé quel que soit le cas envisagé et quelle que soit la température choisie, de -20°C à +150°C, même dans le cas où l'agent acylant est utilisé en excès molaire.

De plus, il est décrit dans EP-0199661 que la diacylation du biphényle dans HF, $BF_3$ à température au moins égale à 50°C. En particulier la diacylation est absolument nulle à 20°C.

La présente invention permet d'éviter un tel choix et conduit à des produits de formule (II) qui ont une pureté élevée, avec un très fort rendement chimique ou rendement de diacylation du composé de formule générale (I)

Elle consiste en un procédé de diacylation de composés de formule générale (I) en composés de formule générale (II), par mise en contact en milieu anhydre, d'un composé de formule générale (I) avec un agent d'acylation de formule générale (III), caractérisé en ce que cette mise en contact s'effectue à une température comprise entre -40°C et 0°C en présence d'acide fluorhydrique HF, de fluorure de bore $BF_3$ et de 2 moles au moins d'agent acylant de formule générale (III) par mole de composé de formule générale (I).

Le but visé par l'invention, la diacylation d'un composé de formule générale (I) est le plus généralement atteint lorsque 1 à 25 moles de HF et 5 à 20 moles de $BF_3$ sont présentes pour une mole dudit composé.

Le rapport molaire $BF_3$/HF qui convient souvent de préférence est compris entre 10/1 et 0,5/1.

Le rapport molaire agent d'acylation/composé de formule (I) est le plus souvent limité à une valeur n'excédant pas 5/1.

Les chlorures d'acide et les anhydrides d'acide sont les agents d'acylation les plus communément utilisés, en particulier le chlorure d'acétyle et l'anhydride acétique.

La présence d'un solvant dans le milieu d'acylation peut être utile, en particulier pour faciliter la solubilisation du composé de formule générale (I) et le contact entre les composants de ce milieu.

Le solvant est alors choisi parmi ceux connus pour convenir dans les réactions de Friedel-Crafts comme par exemple le dichlorométhane, le nitrométhane ou le disulfure de carbone.

La réalisation du procédé de l'invention s'effectue dans tout équipement résistant à la pression et à HF, $BF_3$ dans les conditions définies ci-dessus, comme un autoclave en acier inoxydable.

L'opération selon l'invention est le plus généralement rapide. Sa durée est par exemple de l'ordre de 1 heure, voire moins.

Les exemples suivants, donnés à titre indicatif mais non limitatif, illustrent l'invention.

Dans tous ces exemples,

. le composé de formule générale (I), l'agent d'acylation, HF, $BF_3$ et le cas échéant le chlorure de méthylène employé comme solvant, sont mis en contact dans un autoclave agité en acier inoxydable pendant 1 heure,

. on entend par pureté celle, exprimée en % en poids, du composé de formule générale (II) qui résulte d'une addition d'eau et d'hydroxyde de sodium une fois l'autoclavation achevée, suivie d'une filtration avec lavage à l'eau et d'un séchage sous vide à 80°C,

. on entend par rendement chimique le nombre de moles, exprimé en %, composé de formule générale (I) transformé en composé de formule générale (II).

. Les méthodes d'analyse mises en oeuvre sont la chomatographie en phase liquide et la résonance magnétique nucléaire.

Exemple 1 :

Le diphényléther :

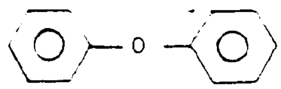

est mis en contact à -40°C avec, par mole de diphényléther, 2,2 moles d'anhydride acétique, 10 moles de HF et 15 moles de $BF_3$.

La pureté du diacétyl-4,4'-diphényléther obtenu, comme le rendement chimique sont supérieurs à 98 %.

Exemple 2 :

Le diphénylsulfure :

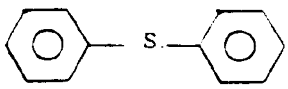

est mis en contact à 0°C avec, pour une mole de diphénylsulfure, 3 moles de chlorure d'acétyle, 10 moles de HF, 7,5 moles de $BF_3$ et 200 g. de chlorure de méthylène.

La pureté du diacétyl-4,4'-diphénylsulfure obtenu est égale à 96 % et le rendement chimique égal à 90%.

Exemple 3 :

Le p.diphénylbenzène :

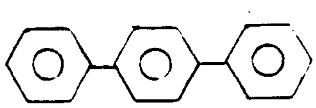

est mis en contact à 0°C avec, pour une mole de p.phénylbenzène, 3 moles de chlorure d'acétyle, 20 moles de HF, 15 moles de $BF_3$ et 400 g. de chlorure de méthylène.

La pureté du diacétyl-4,4'-diphénylbenzène obtenu est supérieure à 98 % et le rendement chimique est égal à 92 %.

Exemple 4 :

Le diphénylméthane :

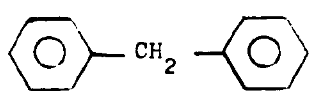

est mis en contact à -20°C avec, pour une mole de diphénylméthane, 2,2 moles d'anhydride acétique, 2,5 moles de HF, 15 moles de $BF_3$ et 200 g. de chlorure de méthylène.

La pureté du diacétyl-4,4'-diphénylméthane obtenu est égale à 90 % et le rendement chimique égal à 20 %.

Exemple 5 :

Le m. benzyltoluène :

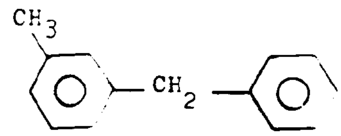

est mis en contact à 0°C avec, pour 1 mole de m.benzyltoluène, 3 moles de chlorure d'acétyle, 2,5 moles de HF et 15 moles de $BF_3$.

Le rendement chimique est de 80 % et le produit obtenu a une pureté de 90 % en composés diacétylés en particulier en position 4 et 4'.

## Revendications

1. Procédé de diacylation de composés ayant deux noyaux aromatiques dans leur molécule de formule générale :

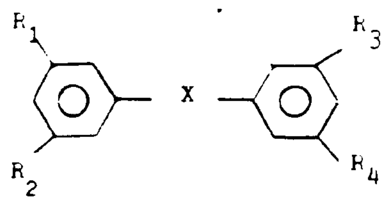

(I),

dans laquelle

X désigne un atome d'oxygène ou de soufre, un groupe

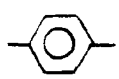

ou $-CH_2-$,

$R_1$, $R_2$, $R_3$, $R_4$, identiques ou différents, représentent un atome d'hydrogène ou un groupe alkyl ayant de 1 à 4 atomes de carbone, en composés de formule générale :

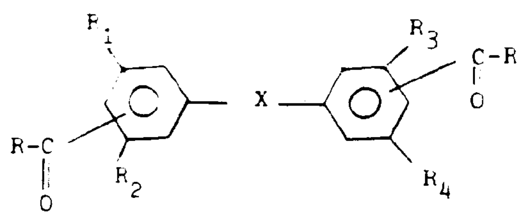

(II),

dans laquelle

X, $R_1$, $R_2$, $R_3$, $R_4$ sont définis comme dans (I),

R est défini comme $R_1$, $R_2$, $R_3$, $R_4$,

à l'aide d'un agent d'acylation de formule générale

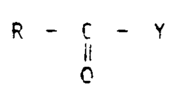

(III),

dans laquelle

Y est un atome d'halogène, un groupe -OH, -OR ou

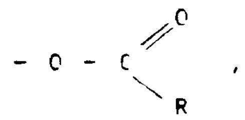

R étant chaque fois défini comme dans (II), par mise en contact en milieu anhydre d'un composé de formule générale (I) avec un agent d'acylation de formule générale (III), caractérisé en ce que cette mise en contact s'effectue à une température comprise entre -40°C et 0°C en présence d'acide fluorhydrique HF, de trifluorure de bore $BF_3$ et de 2 moles au moins d'agent acylant de formule générale (III) par mole de composé de formule générale (I).

2. Procédé selon la revendication 1, caractérisé en ce que 1 à 25 moles de HF et 5 à 20 moles de $BF_3$ sont présentes par mole de composé de formule générale (I).

3. Procédé selon l'une des revendications 1 et 2, caractérisé en ce que le rapport molaire $BF_3$/HF est compris entre 10/1 et 0,5/1.

4. Procédé selon l'une des revendications 1 à 3, caractérisé en ce que le rapport molaire agent d'acylation/composé de formule (I) n'excède pas 5/1.

5. Procédé selon l'une des revendications 1 à 4, caractérisé en ce que l'agent d'acylation est le chlorure d'acétyle.

6. Procédé selon l'une des revendications 1 à 4, caractérisé en ce que l'agent d'acylation est l'anhydride acétique.

7. Procédé selon l'une des revendications 1 à 6, caractérisé en ce que le milieu anhydre comprend un solvant.

8. Procédé selon la revendication 7, caractérisé en ce que le solvant est le chlorure de méthylène.

**Patentansprüche**

1. Verfahren zur Diacylierung von Verbindungen mit zwei aromatischen Kernen in ihrem Molekül der allgemeinen Formel:

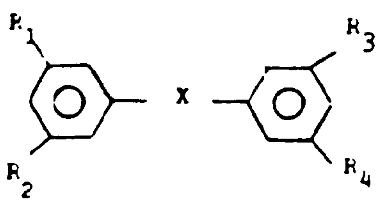

(I)

worin

X ein Sauerstoff- oder Schwefelatom, eine

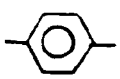

oder eine $CH_2$ Gruppe bezeichnet,

$R_1$, $R_2$, $R_3$, $R_4$ identisch oder unterschiedlich sind und ein Wasserstoffatom oder eine Alkylgruppe mit 1 bis 4 Kohlestoffatomen in Verbindungen der allgemeinen Formel darstellen:

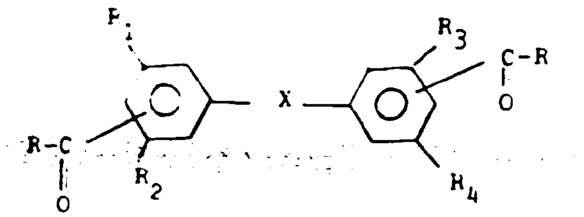

(II)

worin

X, $R_1$, $R_2$, $R_3$, $R_4$ wie in (I) definiert sind,

R wie $R_1$, $R_2$, $R_3$, $R_4$ definiert ist,

mit Hilfe eines Acylierungsmitteis der allgemeinen Formel

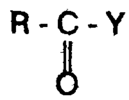

(III)

worin

Y ein Halogenatom, eine OH-, OR- oder folgende Gruppe ist

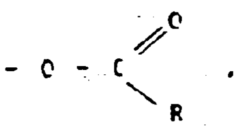

und R jedesmal wie in (II) definiert ist, das darin besteht, daß eine Verbindung der allgemeinen Formnel (I) mit einem Acylierungsmittel der allgemeinen Formel (III) in einem wasserfreien Medium in Berührung gebracht wird, dadurch gekennzeichnet, daß das Inberührungbringen bei einer Temperatur zwischen -40°C und 0°C im Beisein von Fluorwasserstoffsäure HF, von Bortrifluorid $BF_3$ und von mindestens 2 Mol Acylierungsmittel der allgemeinen Formel (III) je Mol Verbindung der allgemeinen Formel (I) Stattfindet.

2. Verfahren nach Anspruch 1, dadurch gekennzeichnet, daß 1 bis 25 Mol HF und 5 bis 20 Mol $BF_3$ je Mol Verbindung der allgemeinen Formel (I) vorhanden sind.

3. Verfahren nach einem der Ansprüche 1 und 2, dadurch gekennzeichnet, daß das Molverhältnis $BF_3$/HF zwischen 10/1 und 0,5/1 beträgt.

4. Verfahren nach einem der Ansprüche 1 bis 3, dadurch gekennzeichnet, daß das Molverhältnis Acylierungsmittel/verbindung der allgemeinen Formel (I) nicht über 5/1 beträgt.

5. Verfahren nach einem der Ansprüche 1 bis 4, dadurch gekennzeichnet, daß das Acylierungsmittel das Azetylchlorid ist.

6. Verfahren nach einem der Ansprüche 1 bis 4, dadurch gekennzeichnet, daß das Acylierungsmittel das Essigsäureanhydrid ist.

7. Verfahren nach einem der Ansprüche 1 bis 6, dadurch gekennzeichnet, daß das wasserfreie Medium ein Lösungsmittel enthält.

8. Verfahren nach Anspruch 7, dadurch gekennzeichnet, daß das Lösungsmittel das Methylenchlorid ist.

## Claims

1. Process for the diacylation of compounds having two aromatic rings in their molecule, of general formula:

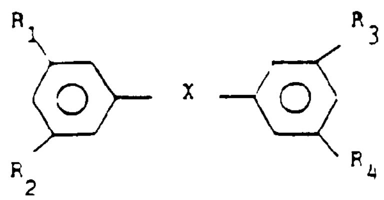 (I).

wherein

X denotes an oxygen or sulphur atom, or a 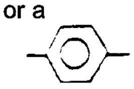 or $-CH_2-$ group,

$R_1$, $R_2$, $R_3$, $R_4$ are identical or different and represent a hydrogen atom or an alkyl group having 1 to 4 carbon atoms, to compounds of general formula:

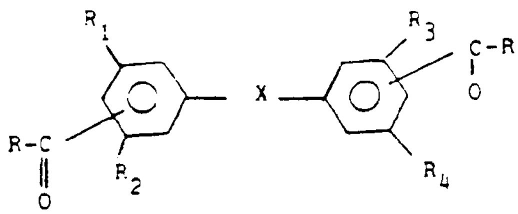 (II),

wherein

X, $R_1$, $R_2$, $R_3$, $R_4$ are definited as in (I),

R is defined as $R_1$, $R_2$, $R_3$, $R_4$,

by means of an acylating agent of general formula:

(III) 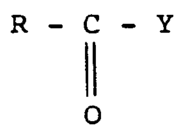

Y is a halogen atom, an -OH or -OR group or

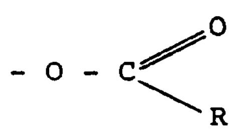

R being defined in each case as in (II), by bringing a compound of general formula (I) into contact with an acylating agent of general formula (III) in anhydrous medium, characterized in that this bringing into contact is effected at a temperature between -40°C and 0°C in the presence of hydrofluoric acid HF, boron trifluoride $BF_3$ and at least 2 moles of acylating agent of general formula (III) per mole of compound of general formula (I).

2. Process according to Claim 1, characterized in that 1 to 25 moles of HF and 5 to 20 moles of $BF_3$ are present per mole of compound of general formula (I).

3. Process according to one of Claims 1 and 2, characterized in that the $BF_3$/HF molar ratio is between 10/1 and 0.5/1.

4. Process according to one of Claims 1 to 3, characterized in that the molar ratio of acylating agent to compound of formula (I) does not exceed 5/1.

5. Process according to one of Claims 1 to 4, characterized in that the acylating agent is acetyl chloride.

6. Process according to one of Claims 1 to 4, characterized in that the acylating agent is acetic anhydride.

7. Process according to one of Claims 1 to 6, characterized in that the anhydrous medium includes a solvent.

8. Process according to Claim 7, characterized in that the solvent is methylene chloride.